# FASCICULE DE BREVET EUROPEEN

(11) **EP 0 545 477 B1**
(45) Date de publication et mention de la délivrance du brevet: **22.03.1995**
(21) Numéro de dépôt: 92203655.3
(22) Date de dépôt: 26.11.1992
(51) Int. Cl.: C07C 67/26

(54) **Procédé de fabrication d'un ester glycidique à partir de glycidol ou dérivé**
Verfahren zur Herstellung eines Glycidylesters aus Glycidol oder eines seiner Derivate
Process for the production of a glycidic ester from glycidol or its derivative

(30) Priorité: 04.12.1991 FR 9115117
(43) Date de publication de la demande: 09.06.1993
(73) Titulaire: ORGANISATION NATIONALE INTERPROFESSIONNELLE DES OLEAGINEUX- ONIDOL, 75008 Paris (FR)
(72) Inventeur: Jamet, Jean-Paul, F-75012 Paris (FR); Parmentier, Jacques, F-60450 Pierrefonds (FR); Vermeersch, Georges, F-75015 Paris (FR); Mouloungui, Zéphirin, F-31400 Toulouse (FR); Rakotondrazafy, Vony, F-31500 Toulouse (FR); Asdih, Mostafa, F-31100 Toulouse (FR); Gaset, Antoine, F-31000 Toulouse (FR); Delmas, Michel, F-31320 Auzeville Tolosane (FR); Anuku, Nicolas, F-31100 Toulouse (FR)
(74) Mandataire: Cabinet BARRE LAFORGUE & associés

(56) Documents cités:
- BE-A- 657 517
- US-A- 3 251 870
- US-A- 3 404 174
- US-A- 4 970 333

## Description

L'invention concerne un procédé de fabrication d'un ester glycidique à partir de glycidol ou dérivé de glycidol comportant un cycle époxy 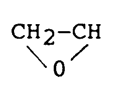
et un oxygène sur le radical latéral fixé au groupe époxy. L'invention vise en particulier à fabriquer des glycérides : monoglycérides tels que monooléate, monostéarate, monoundécylénoate de glycérol... ou di ou triglycérides tels que dioléate ou trioléate, distéarate ou tristéarate de glycérol...

Les esters glycidiques et plus particulièrement les glycérides sont des composés utilisés dans plusieurs secteurs industriels : industries alimentaires et cosmétologie (émulsifiant, épaississant, gélifiant), industries pharmaceutiques (anti-inflammatoire, antibactérien, analgésique...), industries textiles (enrobage de fibres), peintures (émulsifiant et lubrifiant), surfactants...

Traditionnellement, la fabrication des glycérides s'effectue par voie chimique par une estérification d'un acide gras ou une transestérification d'un triglycéride par le glycérol ou un dérivé de celui-ci (DE 3.909.097, DE 3.020.566, JP 1.117.845...). Ces réactions qui utilisent comme produit de départ un polyol : le glycérol (ou dérivé), consistent à remplacer l'hydrogène d'un ou plusieurs groupements hydroxyle du glycérol par un ou des groupements acyle provenant de l'acide gras ou du triglycéride. Les défauts essentiels de ces réactions résident dans la sévérité des conditions opératoires (température de réaction élevée généralement supérieure à 130° C, pression élevée supérieure à la pression atmosphérique, atmosphère inerte), dans la nature des catalyseurs utilisés et leurs conditions d'emploi (catalyseurs en phase homogène, difficiles à extraire et à recycler) et dans la mauvaise sélectivité et le rendement souvent médiocre des réactions.

Un autre type de procédés par voie chimique consiste à fabriquer les glycérides à partir d'un autre composé de départ : le glycidol ou un dérivé qui comporte un cycle époxy 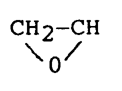
et un oxygène sur le radical latéral fixé au groupe époxy. Le procédé de l'invention vise un procédé de ce type.

Dans les procédés connus utilisant comme composé de départ le glycidol, l'on met un acide gras en présence d'un ou plusieurs catalyseurs en phase homogène afin d'engendrer une attaque de cet acide et la formation d'un carboxylate. Ce dernier réagit ensuite avec le glycidol pour ouvrir son cycle époxy et se fixer sur celui-ci. Les catalyseurs utilisés sont généralement des halogénures de tétra éthyl ammonium ou un mélange de l'un de ces composés avec un alcoolate ou un alcoxyde de métal (Na, Ti, Ta, Al...) (références : US 2.910.490 ; Von Dr. Ernst Ulsperger, Tenside, vol. 2, 1965, pages 331-337 ; S.N. Zlatanos et al, JAOCS, vol. 62, n° 11, novembre 1985, pages 1575-1577 ; Maurice Caron, K.B. Sharpless, J. Org. Chem., 1985, pages 1557-1560 ; Carmen E. Burgos et al, J. Org. Chem., vol. 52, 1987, pages 4973-4977). Ce procédé se caractérise donc par la formation d'un composé intermédiaire : un carboxylate qui réagit ensuite avec le glycidol. Toutefois, la formation de ce composé intermédiaire exige des conditions contraignantes très spécifiques : nature des catalyseurs et des solvants (toxiques) et conditions d'utilisation en grande quantité, en phase homogène, donc difficiles à extraire, température relativement élevée (de l'ordre de 110° à 160°), nécessité de travailler avec un large excès de glycidol ce qui implique son extraction en fin de réaction ; en outre, dans les meilleures conditions opératoires, ce procédé présente un rendement inférieur à 90 % et conduit à des produits de pureté inférieure à 90 %.

Il est à noter que, dans d'autres secteurs de la chimie, sont connus des procédés consistant à former préalablement un carboxylate qui sert de composé auxiliaire pour ouvrir les cycles époxy d'un réactif de départ. Par exemple, les brevets US 4.970.333 et BE 657.517 décrivent un procédé de fabrication d'ester de glycol (monoester hydroxy-alkyl) à partir d'oxyde d'alkylène, qui consiste à faire réagir une résine anionique dopée par des radicaux acryliques avec un acide pour engendrer la formation intermédiaire d'un carboxylate, et à utiliser ce dernier pour ouvrir les cycles époxy de l'oxyde d'alkylène, en vue de conduire à l'ester d'alkylène glycol. Cette réaction s'effectue sous pression et la résine utilisée agit comme réactif et est consommée. Ce type de procédé ne fournit aucun enseignement qui permettrait d'améliorer les procédés précités de fabrication de glycéride à partir de glycidol et vient simplement confirmer qu'il est possible d'ouvrir un cycle époxy au moyen d'un carboxylate, afin de le faire réagir ensuite avec un autre composé.

Enfin, il existe une voie plus récente de fabrication des monoglycérides : la voie enzymatique qui permet de transformer des triglycérides ou acides gras en monoglycérides par transestérification ou estérification. La transestérification enzymatique est essentiellement intéressante pour obtenir l'isomère béta.

Par ailleurs, un brevet chinois n° 87.107970.4 décrit une technique de transestérification de triglycéride par un alcool, qui conduit à un mélange de monoglycéride et d'ester d'acide gras. Cette technique utilise comme catalyseur une résine anionique sous forme OH⁻ afin de former un composé intermédiaire : un alcoolate qui réagit ensuite sur le triglycéride pour opérer la transestérification. Toutefois, cette technique utilise comme composé de départ un triglycéride et permet simplement de le transestérifier en monoglycéride ; elle implique donc que le triglycéride ait été préalablement préparé, alors que l'invention vise la fabrication de glycéride (et plus largement d'ester glycidique) à partir de composés de départ de familles différentes : glycidol ou dérivé et acide carboxylique. Il est à noter que cette réaction de transestérification du triglycéride conduit à un mélange de produits (monoglycéride et ester d'acide gras) et présente un rendement en monoglycéride qui ne dépasse pas 70 %.

La présente invention se propose d'améliorer les procédés de fabrication de glycéride (et plus généralement d'ester glycidique) utilisant comme composés de départ le glycidol ou un dérivé et un acide carboxylique.

L'objectif de l'invention est d'indiquer une nouvelle réaction pour la fabrication d'esters glycidiques, se caractérisant par :
. les composés de départ constitués par du glycidol (ou dérivé possédant un cycle époxy et une chaîne latérale dotée d'un oxygène) et un acide carboxylique,
. l'utilisation d'un catalyseur non toxique intégralement recyclable,
. une mise en oeuvre en milieu hétérogène facilitant l'extraction du catalyseur,
. une température de mise en oeuvre basse ou modérée (en particulier de la température ambiante jusqu'à 70° C) et une pression de travail pouvant être la pression atmosphérique,
. une sélectivité à l'égard de l'ester glycidique très élevée,
. une absence d'isomérisation, la forme cis ou trans de l'acide de départ étant conservée.

Le procédé de l'invention pour la fabrication d'un ester glycidique à partir de glycidol ou dérivé de glycidol et d'acide carboxylique, se caractérise en ce que le glycidol ou dérivé et l'acide carboxylique sont mis en présence d'un catalyseur solide constitué par une résine anionique forte macroporeuse sous forme OH⁻ de façon à assurer l'ouverture des cycles époxy par l'action des sites actifs OH⁻ de la résine, et à permettre une attaque de l'acide carboxylique sur ces cycles époxy ouverts, conduisant à la fixation dudit acide sur les cycles avec libération de l'ester glycidique et régénération de la résine sous forme OH⁻.

Ainsi, dans le procédé de l'invention, l'ester glycidique est obtenu sans formation d un composé intermédiaire (en particulier en l'absence de carboxylate dont la formation requiert des conditions sévères et est un facteur de chute du rendement). Dans l'invention, le mécanisme réactionnel totalement nouveau est le suivant : le cycle époxy du glycidol est ouvert par les sites actifs de la résine, l'acide carboxylique demeurant inerte vis-à-vis de la résine ; cet acide se fixe ensuite sur le carbone alpha du cycle ouvert sous forme d'ester. Ainsi, le catalyseur engendre l'ouverture du cycle époxy mais n'est pas consommé et retrouve sa forme OH⁻ à la fin des interactions.

La réaction peut être mise en oeuvre en milieu aqueux, ou dans un milieu organique inerte à l'égard des composés présents, ou même dans un excès d'acide carboxylique.

Le solvant organique, aqueux ou carboxylique, favorise la formation des liaisons hydrogènes entre les réactifs et les sites actifs de la résine (sous forme de complexe accepteur/donneur) ; ces liaisons hydrogènes sont responsables de l'activation anionique observée qui conduit à l'ouverture des cycles époxy et à la fixation de l'acide.

Le milieu aqueux permet, le cas échéant, d'utiliser une résine anionique conditionnée sous forme de sel (chlorure ou autre), ledit milieu aqueux fournissant à celle-ci les sites OH⁻ par substitution anionique ; toutefois, le rendement est alors moins bon.

Le procédé de l'invention peut être mis en oeuvre à température ambiante et à pression atmosphérique. En pratique, l'on choisira une température comprise entre 20° C et 70° C (un léger apport thermique augmente sensiblement la cinétique de la réaction).

Le catalyseur se présentant sous forme solide, l'ester glycidique peut être aisément extrait dans un état de pureté élevé compte-tenu de la bonne sélectivité de la réaction (100 % vis-à-vis de l'ester).

Les essais ont permis de constater une absence complète d'isomérisation en cours de réaction de sorte que, par un choix de l'acide de départ (mono ou polyinsaturé), l'on peut obtenir l'isomère d'ester désiré.

Le procédé peut être mis en oeuvre en discontinu en mélangeant la résine et la phase liquide (glycidol, acide, milieu aqueux ou organique) dans un réacteur et en séparant par filtration la résine et l'ester obtenu ; il peut être également mis en oeuvre en continu en amenant la phase liquide à percoler à travers un lit de résine disposé dans une colonne de réaction.

Le procédé de l'invention peut en particulier s'appliquer pour fabriquer un glycéride à partir de glycidol et d'acide gras. L'on a pu constater dans ce cas que le choix du milieu permet d'orienter la sélectivité vers le monoglycéride ou vers les diglycérides et/ou triglycérides. Ainsi, les éthers polyoxygénés (1,4-dioxane, diglyme, triglyme...), l'eau, l'acide carboxylique, l'acétate d'éthyle et l'acétone favorisent la formation de monoglycéride (sélectivité proche de 100 %) alors que les solvants hydrocarbonés (hexane, toluène, benzène...) favorisent plutôt la formation des diglycérides et/ou triglycérides.

Le procédé de l'invention permet également de fabriquer d'autres esters, par exemple acrylate furanique de glycérol à partir de glycidol et d'un acide acrylique furanique.

Le procédé peut également être appliqué pour fabriquer des esters glycidiques éthérés en utilisant un éther de glycidol et un acide gras.

Le procédé de l'invention est avantageusement mis en oeuvre dans les conditions opératoires suivantes, qui améliorent la sélectivité de la réaction, son rendement et la pureté de l'ester obtenu :
. l'on dispose la résine anionique de sorte que le nombre de sites actifs OH⁻ exprimé en milliéquivalent OH par millimole de glycidol ou dérivé soit compris entre 0,2 et 1,
. l'on utilise une résine anionique dont la capacité est comprise entre 0,8 et 3 milliéquivalents OH par gramme.

Des essais comparatifs de différentes résines disponibles sur le marché ont permis de constater que les meilleurs résultats semblaient être fournis par les résines anioniques suivantes : "Lewatit MP 500" fabriquée par la Société "Bayer", "Amberlyst A 26" fabriquée par la Société "Rohm and Haas".

De plus, en cas d'utilisation d'un milieu aqueux ou d'un milieu organique inerte, l'on mélange avantageusement le glycidol ou dérivé et l'acide carboxylique de sorte que la concentration de chacun de ces composés soit comprise entre 0,1 et 1 mole de composé par litre de milieu.

Dans le cas où la réaction est mise en oeuvre dans un excès d'acide carboxylique, l'on effectue avantageusement le mélange de sorte que la concentration de glycidol ou dérivé soit compris entre 0,2 et 0,7 mole de composé par litre de milieu.

Bien entendu, le procédé de l'invention peut, le cas échéant, être mis en oeuvre en utilisant comme corps de départ un mélange d'acides carboxyliques (par exemple certaines huiles d'acidité faible, moyenne ou forte disponibles sur le marché) ; on obtient alors un mélange des esters correspondants (mélange qui peut être utilisé comme huile neutre).

Le procédé de l'invention peut être mis en oeuvre en utilisant directement le glycidol ou dérivé comme corps de départ ; il est également possible, dans une phase préalable, de fabriquer celui-ci in situ à partir d'une monohalohydrine ; c'est là un avantage supplémentaire de l'invention, car cette famille de composés est d'un accès facile et peu onéreux ; par exemple l'on peut utiliser l'α-monochlorhydrine. La phase préalable consiste alors à effectuer dans le réacteur ou la colonne de réaction contenant la résine anionique une deshydrohalogénation de la monohalohydrine en milieu aqueux ; cette réaction est connue en soi et on pourra par exemple se reporter au brevet BE 554.639 qui la décrit.

Les exemples qui suivent illustrent le procédé de l'invention et ses performances, et le dessin schématise le mécanisme réactionnel. Les rendements indiqués en ester glycidique sont définis comme le rapport entre le nombre de moles d'ester glycidique obtenues et le nombre de moles de glycidol initial.

### Exemple 1 :

Cet exemple vise la fabrication de monooléate de glycérol à partir de glycidol et d'acide oléique.

Dans un réacteur de 250 ml, l'on mélange simultanément :
. 16 mmoles de glycidol,
. 16 mmoles d'acide oléique (isomère cis),
. 25 mlitres de 1,4-dioxane,
. 5 g de résine anionique "Lewatit MP 500" sous forme OH⁻, de capacité de 2,4 milliéquivalents OH par gramme.

Ces proportions correspondent à une concentration de glycidol ou d'acide dans le solvant égale à 0,6 mole/litre.

De plus, le nombre de sites actifs OH⁻ de la résine est égal à 0,75 milliéquivalent par millimole de glycidol.

Le mélange est porté à 45° C et agité pendant 6 heures. Le suivi de la réaction est réalisé par chromatographie sur couche mince en prélevant des échantillons toutes les heures. Au bout de 6 heures, la réaction est totale (absence complète de glycidol et d'acide oléique).

L'on filtre ensuite le mélange pour séparer la phase solide et la phase liquide.

La phase solide est lavée, puis analysée par dosage classique acidobasique ; l'on constate que l'on retrouve la résine de départ sous la même forme avec sensiblement la même capacité de 2,4 milliéquivalents OH/g. Il est à noter toutefois qu'une réutilisation de la résine dans plusieurs cycles de fabrication entraîne une chute progressive de capacité. En pratique, la même résine peut être utilisée 5 fois avant d'être régénérée à la soude.

La phase liquide est concentrée par évaporation et l'on obtient un résidu cristallisé blanc qui est analysé (R.M.N. du proton, du carbone 13, analyse infrarouge, microanalyse...). Ce résidu est exclusivement du monooléate de glycérol (sélectivité : 100 %) de formule : 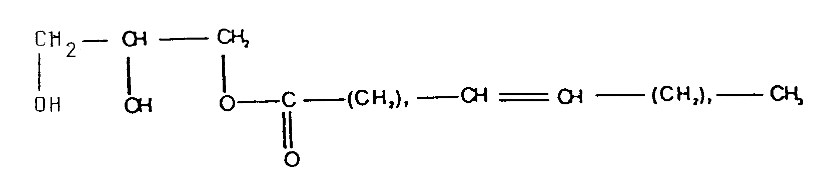
La quantité récupérée est de 15,7 millimoles, soit un rendement en monoléate de 98 %.

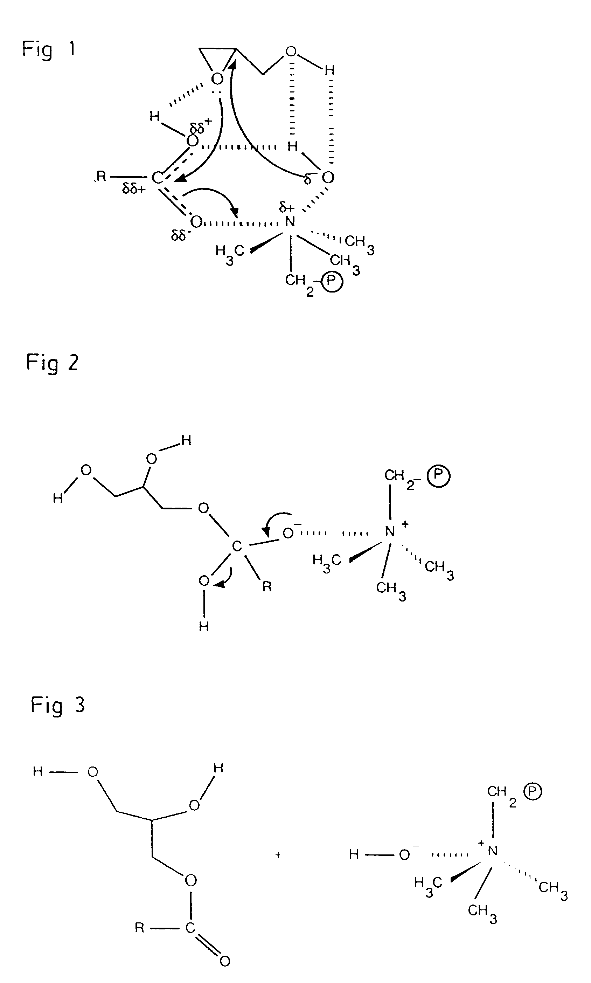

Les études ont montré que le mécanisme réactionnel pouvait être schématisé comme représenté aux figures 1, 2 et 3.

La figure 1 illustre l'activation des réactifs par les sites actifs de la résine.

R représente la chaîne grasse de l'acide gras et P la matrice de polystyrène.

Les composés sont dans un état de transition grâce aux phénomènes de chimisorption conditionnés par les liaisons hydrogènes. Le solvant polyoxygéné (dioxane) favorise l'établissement de ces liaisons donneur/accepteur, conduisant à l'adsorption du glycidol et de l'acide carboxylique sur les sites de la résine.

La figure 2 illustre la décomposition de l'agrégat obtenu dans l'état de transition, conduisant à l'ouverture de l'époxyde et à l'attaque de carbone du carbonyle ce l'acide carboxylique.

La figure 3 illustre la formation du monoglycéride et la régénération de la résine sous forme OH⁻ au terme du processus.

### Exemple 2 :

Cet exemple vise la fabrication du monooléate de glycérol dans un solvant différent de celui de l'exemple 1.

Les conditions opératoires sont les mêmes que précédemment, exceptée l'utilisation du triglyme comme solvant de réaction à la place du dioxane.

Le mélange réactionnel final est filtré pour séparer la résine de la phase liquide.

On ne peut évaporer le solvant dont le point d'ébullition est élevé.

La phase liquide est distillée de façon à éliminer le triglyme pour obtenir un mélange final contenant du monooléate de glycérol, avec un rendement de 58 %.

### Exemple 3 :

Cet exemple vise la fabrication de monostéarate de glycérol.

Dans les mêmes conditions opératoires que l'exemple 1, l'on obtient avec l'acide stéarique comme acide gras de départ, exclusivement le monostéarate de glycérol.

Au bout de la même durée de réaction, il est obtenu avec un rendement de 74 %.

### Exemple 4 :

Cet exemple vise la fabrication du monostéarate de glycérol dans un solvant différent (triglyme).

Les opérations se déroulent de la même manière que l'exemple 2 et l'on prend à la place de l'acide oléique, l'acide saturé correspondant : acide stéarique.

L'on observe le même comportement et l'on obtient un mélange final contenant du monostéarate de glycérol, avec un rendement de 51 %.

### Exemple 5 :

Cet exemple vise la fabrication de monoundécylénoate de glycérol.

Avec l'acide undécylénique, les conditions opératoires de l'exemple 1 conduisent à l'obtention de façon sélective du monoglycéride de l'acide utilisé. La réaction est quasi-totale et l'on récupère 15,6 mmoles de produit final, soit un rendement de 98 % en monoundécylénoate de glycérol.

### Exemple 6 :

Cet exemple vise la fabrication de monoundécylénoate de glycérol avec un solvant différent (triglyme).

Reprenant les mêmes conditions opératoires que l'exemple 2, et remplaçant l'acide oléique par l'acide undécylénique, l'on constate le même comportement du milieu réactionnel qui, au bout de 6 heures, contient du monoundécylénoate de glycérol, avec un rendement de 53 %.

### Exemple 7 :

Cet exemple vise la fabrication du monooléate de glycérol utilisant :
- la résine anionique A26 OH "Amberlyst-26 de Rohm and Haas" de capacité 2,4 milliéquivalents OH par gramme à la place de la résine "Lewatit MP 500", dans la même concentration,
- l'hexane comme solvant de réaction,
- et un rapport acide/glycidol de 1/_{0,25}.

L'on obtient au bout de 6 heures un mélange final contenant du monooléate de glycérol, avec un rendement de 46 %. La sélectivité est de 100 % à l'égard du composé.

### Exemple 8 :

Cet exemple vise la fabrication de trioléate de glycérol dans un autre solvant (benzène).

Par rapport à l'exemple 1, lorsqu'on remplace le dioxane 1,4 par le benzène, on constate la formation de produits majoritaires tels que le mono et le trioléate de glycérol en proportions sensiblement identiques. On remarque la présence de traces de 1,2 dioléate et 1,3 dioléate de glycérol. La sélectivité au bout de 6 heures de réaction est de 99 %.

### Exemple 9 :

Cet exemple vise la fabrication de tristéarate de glycérol.

En reprenant l'exemple 7 avec l'acide stéarique comme acide gras de départ, l'on synthétise essentiellement du tristéarate de glycérol avec des traces de mono et de distéarates de glycérol. Le rendement en ester glycérique s'élève à 98 %.

### Exemple 10 :

Cet exemple vise la fabrication de 3-(2-furyl) propénoate de glycérol à partir de l'acide 3-(2-furyl) acrylique.

Les conditions opératoires de l'exemple 1 reprises avec l'acide 3-(2-furyl) acrylique à la place de l'acide oléique, avec un rapport molaire acide carboxylique/glycidol égal à 1,5, conduisent à l'obtention de façon sélective de l'ester correspondant au bout de 7,5 heures de réaction. Le rendement s'élève à 70 % en 3-(2-furyl) propénoate de glycérol.

### Exemple 11 :

Cet exemple vise la fabrication du monooléate de glycérol.

Reprenant les conditions opératoires de l'exemple 1, et remplaçant la résine anionique "Lewatit MP 500" par la résine A26 OH, et le solvant par de l'acétone, l'on constate la formation sélective du monooléate de glycérol au bout de 6 heures avec un rendement de 28 %. La sélectivité est de 100 %.

### Exemple 12 :

Cet exemple vise la fabrication du monooléate de glycérol.

Avec les mêmes conditions opératoires que l'exemple 11, exceptée l'utilisation de l'acétate d'éthyle comme solvant de réaction, l'on obtient pour une durée de 6 heures un mélange final contenant du monooléate de glycérol, avec un rendement de 39 %. La sélectivité est de 100 %.

### Exemple 13 :

Cet exemple vise la fabrication du monooléate de glycérol.

Dans les mêmes conditions opératoires que l'exemple 12, et en remplaçant le solvant de réaction par l'acide oléique technique (60-80 % d'acide oléique), l'on forme au bout de 6 heures du monooléate de glycérol avec 44 % de rendement. La sélectivité est de 100 %.

### Exemple 14 :

Cet exemple vise la fabrication du monooléate de glycérol.

En présence de la résine A26 OH, utilisant un nombre de sites actifs égal à 0,50 milliéquivalent par millimole d'acide oléique et pour une proportion molaire acide/glycidol de 1/_{0,25}, l'on forme au bout de 6 heures, en milieu aqueux, du monooléate de glycérol, avec un rendement de 20 %. Le milieu aqueux est réalisé de sorte que la concentration de l'acide carboxylique soit égale à 0,8 mole par litre de milieu, et la concentration de glycidol égale à 0,2 mole par litre de milieu.

### Exemple 15 :

Cet exemple vise la fabrication du monooléate de glycérol.

Reprenant les mêmes conditions que l'exemple 14, exceptée l'utilisation de la résine A26 sous forme Cl, l'on obtient un rendement de 5 % en monooléate de glycérol. La forme OH de la résine est donc importante dans ce cas pour obtenir un bon rendement.

### Exemple 16 :

Cet exemple vise la fabrication de monooléate de glycérol.

Dans une phase préalable, l'on dispose dans un réacteur contenant 100 millilitres d'eau (température ambiante, pression atmosphérique) :
- 3,8 millimoles d'α-monochlorhydrine,
- 11,6 g de résine anionique A26 OH de capacité égale à 2,63 milliéquivalents OH⁻ par gramme de résine sèche.

Au bout de 6 cycles d'une durée totale de 12 minutes, l'on dose le glycidol et l'on constate que 75 % de l'α-monochlorhydrine s'est transformé en ce composé avec une sélectivité de 100 %.

L'on ajoute alors dans le réacteur 74 millimoles d'acide oléique.

Cette proportion permet de revenir aux conditions de l'exemple 15.

### Exemple 17 :

Cet exemple vise la fabrication du monooléate de glycérol dans les mêmes conditions que l'exemple 16, mais sur colonne de résine (30 cm de hauteur, 2,5 cm de diamètre, masse de résine : 11,6 g) identique à celle de l'exemple précédent.

On amène à percoler 38 mmoles d'α-monochlorhydrine à travers la colonne de résine. Le glycidol est obtenu avec un rendement de 75 % après 4 cycles de réaction (4 circulations dans la colonne de durée globale égale à 8 minutes). L'on mélange ensuite à la phase liquide obtenue 74 mmoles d'acide oléique et on amène le mélange à percoler dans la colonne de résine. Le monooléate de glycérol est obtenu avec un rendement de 5 % (par rapport au glycidol formé) et une sélectivité de 100 %.

## Revendications

1. Procédé de fabrication d'un ester glycidique du type consistant à utiliser un glycidol ou dérivé de glycidol comportant un cycle époxy 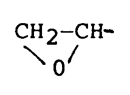 et une chaîne latérale dotée d'un oxygène, et à faire réagir ledit glycidol ou dérivé avec de l'acide carboxylique, ledit procédé étant caractérisé en ce que le glycidol ou dérivé et l'acide carboxylique sont mis en présence d'un catalyseur solide constitué par une résine anionique forte macroporeuse sous forme OH⁻, de façon à assurer l'ouverture des cycles époxy par l'action des sites actifs OH⁻ de la résine, et à permettre une attaque de l'acide carboxylique par ces cycles époxy ouverts, conduisant à la fixation dudit acide sur les cycles avec libération de l'ester glycidique et régénération de la résine sous forme OH⁻.

2. Procédé de fabrication selon la revendication 1, caractérisé en ce que la réaction est mise en oeuvre en milieu aqueux ou dans un milieu organique inerte à l'égard des composés présents.

3. Procédé de fabrication selon la revendication 1, caractérisé en ce que la réaction est mise en oeuvre dans un excès d'acide carboxylique.

4. Procédé selon l'une des revendications 1, 2 ou 3, dans lequel l'on utilise le glycidol et un acide gras pour obtenir un glycéride.

5. Procédé selon la revendication 4 pour la fabrication d'un monoglycéride, caractérisé en ce que l'on mélange le glycidol ou dérivé et l'acide gras dans un solvant constitué par un éther polyoxygéné.

6. Procédé selon la revendication 4 pour la fabrication du monooléate de glycérol à partir de glycidol et d'acide oléique, caractérisé en ce que l'on mélange ces composés dans un solvant du groupe suivant : 1,4-dioxane, acétate d'éthyle, acétone, diglyme ou triglyme.

7. Procédé selon la revendication 5 pour la fabrication d'un monostéarate de glycérol à partir de glycidol et d'acide stéarique, caractérise en ce que l'on mélange ces composés dans un solvant constitué par du 1,4-dioxane ou du diglyme ou triglyme.

8. Procédé selon la revendication 5 pour la fabrication d'un monoundécylénoate de glycérol à partir de glycidol et d'acide undécylénique, caractérisé en ce que l'on mélange ces composés dans un solvant constitué par du 1,4-dioxane ou du diglyme ou triglyme.

9. Procédé selon la revendication 4 pour la fabrication d'un diglycéride ou triglycéride, caractérisé en ce que l'on mélange le glycidol ou dérivé et l'acide gras dans un solvant hydrocarboné.

10. Procédé selon la revendication 9 pour la fabrication de dioléate et de trioléate de glycérol à partir de glycidol et d'acide oléique, caractérisé en ce que l'on mélange ces composés dans un solvant constitué par de l'hexane, du toluène ou du benzène.

11. Procédé selon la revendication 9 pour la fabrication de distéarate et tristéarate de glycérol à partir de glycidol et d'acide stéarique, caractérisé en ce que l'on mélange ces composés dans un solvant constitué par de l'hexane, du toluène ou du benzène.

12. Procédé selon l'une des revendications 1, 2 ou 3, caractérisé en ce que l'on utilise du glycidol et un acide acrylique furanique pour obtenir un acrylate furanique de glycérol.

13. Procédé selon la revendication 1, 2 ou 3 dans lequel l'on utilise un éther de glycidol et un acide gras pour obtenir un ester glycidique éthéré.

14. Procédé selon la revendication 2, caractérisé en ce que l'on met en présence sensiblement 1 mole d'acide carboxylique par mole de glycidol ou dérivé de glycidol.

15. Procédé selon l'une des revendications 2 ou 14, caractérisé en ce que l'on mélange le glycidol ou dérivé et l'acide carboxylique en milieu aqueux ou milieu organique inerte de sorte que la concentration de chacun de ces composés soit comprise entre 0,1 et 1 mole de composé par litre de milieu.

16. Procédé selon la revendication 3, caractérisé en ce que l'on mélange le glycidol ou dérivé et l'acide carboxylique de sorte que la concentration de glycidol ou dérivé soit comprise entre 0,2 et 0,7 mole de composé par litre de milieu.

17. Procédé selon l'une des revendications 1 à 16, caractérisé en ce que l'on dispose la résine anionique de sorte que le nombre de sites actifs OH⁻ exprimé en milliéquivalent OH par millimole de glycidol ou dérivé soit compris entre 0,2 et 1.

18. Procédé selon la revendication 17, caractérisé en ce que l'on utilise une résine anionique dont la capacité est comprise entre 0,8 et 3 milliéquivalents OH par gramme.

19. Procédé selon la revendication 17, caractérisé en ce que l'on utilise une résine anionique du groupe suivant : résine "Lewatit MP 500" fabriquée par la Société "Bayer", résine "Amberlyst A 26" fabriquée par la Société "Rohm and Haas".

20. Procédé selon l'une des revendications 1 à 19, mis en oeuvre à une température comprise entre 20° C et 70° C.

21. Procédé selon l'une des revendications 1 à 20 mis en oeuvre en discontinu dans un réacteur, dans lequel l'on mélange la résine et la solution dans le réacteur et l'on sépare par filtration la résine et l'ester obtenu.

22. Procédé selon l'une des revendications 1 à 20 mis en oeuvre en continu dans une colonne de réaction, dans lequel l'on amène la solution à percoler à travers un lit de résine disposé dans la colonne de réaction.

23. Procédé de fabrication selon l'une des revendications 21 ou 22 dans lequel, dans une phase préalable, l'on fabrique le glycidol ou dérivé, in situ dans le réacteur ou dans la colonne de réaction, en effectuant en milieu aqueux une deshydrohalogénation d'une monohalohydrine en présence de la résine anionique.

24. Procédé de fabrication selon la revendication 23, dans lequel on utilise, dans la première phase, l'α-monochlorhydrine.

## Claims

1. Process for the production of a glycidic ester of the type which consists in using a glycidol or glycidol derivative comprising an epoxy ring 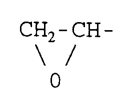 and a side chain endowed with an oxygen atom and in causing said glycidol or glycidol derivative to react with carboxylic acid, said process being characterised in that the glycidol or glycidol derivative and the carboxylic acid are brought together with a solid catalyst constituted by a highly macroporous anionic resin of OH⁻ type so as to ensure, by the action of the active OH⁻ sites of the resin, opening of the epoxy rings and in enabling an attack on the carboxylic acid by said open epoxy rings, whereby said attack leads to the fixation of said acid on said rings accompanied by the release of glycidic ester and regeneration of the resin of OH⁻ type.

2. Production process according to Claim 1, characterised in that the reaction is brought about in aqueous medium or in an organic medium which is inert with respect to the compounds present.

3. Production process according to Claim 1, characterised in that the reaction is brought about in an excess of carboxylic acid.

4. Process according to one of Claims 1, 2 or 3, in which use is made of the glycidol and a fatty acid in order to obtain a glyceride.

5. Process according to Claim 4 for the production of a monoglyceride, characterised in that the glycidol or glycidol derivative is mixed with the fatty acid in a solvent constituted by a polyoxygenated ether.

6. Process according to Claim 4 for the production of glycerol monooleate from glycidol and oleic acid, characterised in that said compounds are mixed in a solvent pertaining to the following group: 1.4-dioxane, ethyl acetate, acetone, diglyme or triglyme.

7. Process according to Claim 5 for the production of a glycerol monostearate from glycidol and stearic acid, characterised in that said compounds are mixed in a solvent constituted by 1.4-dioxane or diglyme or triglyme.

8. Process according to Claim 5 for the production of a glycerol monoundecylenoate from glycidol and undecylenic acid, characterised in that said compounds are mixed in a solvent constituted by 1.4- dioxane or diglyme or triglyme.

9. Process according to Claim 4 for the production of a diglyceride or triglyceride, characterised in that the glycidol or glycidol derivative is mixed with the fatty acid in a hydrocarbon solvent.

10. Process according to Claim 9 for the production of glycerol dioleate and trioleate from glycidol and oleic acid, characterised in that said compounds are mixed in a solvent constituted by hexane, toluene or benzene.

11. Process according to Claim 9 for the production of glycerol distearate and tristearate from glycidol and stearic acid, characterised in that said compounds are mixed in a solvent constituted by hexane, toluene or benzene.

12. Process according to one of Claims 1, 2 or 3, characterised in that glycidol and an acrylofuranic acid are used to obtain a furanic acrylate of glycerol.

13. Process according to Claim 1, 2 or 3, in which use is made of a glycidol ether and a fatty acid in order to obtain an etherified glycidic ester.

14. Process according to Claim 2, characterised by the application of substantially 1 mol carboxylic acid per mol glycidol or glycidol derivative

15. Process according to one of Claims 2 or 14, characterised in that the glycidol or glycidol derivative is mixed with the carboxylic acid in aqueous medium or inert organic medium so that the concentration of each of said compounds amounts to between 0.1 and 1 mol of compound per litre of medium.

16. Process according to Claim 3, characterised in that the glycidol or glycidol derivative is mixed with carboxylic acid so that the concentration of glycidol or glycidol derivative amounts to between 0.2 and 0.7 mol of compound per litre of medium.

17. Process according to one of Claims 1 to 16, characterised in that the anionic resin is prepared in such a way that the number of active OH⁻ sites expressed in OH milliequivalent per millimol glycidol or glycidol derivative amounts to between 0.2 and 1.

18. Process according to Claim 17, characterised in that use is made of an anionic resin the capacity of which amounts to between 0.8 and 3 milliequivalents of OH per gram.

19. Process according to Claim 17, characterised in that use is made of an anionic resin pertaining to the following group: "Lewatit MP 500" resin manufactured by "Bayer", "Amberlyst A 26" resin manufactured by "Rohm and Haas".

20. Process according to one of Claims 1 to 19, carried out at a temperature between 20° C and 70° C.

21. Process according to one of Claims 1 to 20 carried out discontinuously within a reactor, whereby the resin is mixed with the solution in the reactor and the resin and the ester obtained are separated by filtration.

22. Process according to one of Claims 1 to 20, carried out continuously within a reaction column whereby the solution is caused to percolate through a layer of resin arranged within the reaction column.

23. Production process according to one of Claims 21 or 22, in which, during a preliminary stage, the glycidol or glycidol derivative is produced in situ within the reactor or within the reaction column by effecting, in an aqueous medium, dehydrohalogenation of a monohalohydrin in the presence of the anionic resin.

24. Production process according to Claim 23, in which use is made, in the first stage, of α-monochlorohydrin.

## Patentansprüche

1. Verfahren zur Herstellung eines Glycidylesters der Art, die darin besteht, daß von einem Glycidol oder einem Glycidolderivat Gebrauch gemacht wird, das einen Epoxy-Ring 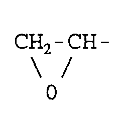 und eine mit einem Sauerstoffatom dotierte Seitenkette umfaßt, sowie darin, daß zwischen dem besagten Glycidol bzw. Glycidolderivat und Carbonsäure eine Reaktion bewirkt wird, wobei das besagte Verfahren dadurch gekennzeichnet ist, daß das Glycidol bzw. Glycidolderivat und die Carbonsäure mit einem festen, aus hochgradig mikroporösem anionischem Harz in der Form OH⁻ bestehenden Katalysator in Kontakt gebracht werden, um sicherzustellen, daß die Epoxy-Ringe unter der Einwirkung der aktiven OH⁻ Stellen des Harzes geöffnet werden, und um Angriff der Carbonsäure durch die besagten offenen Epoxy-Ringe zu ermöglichen, so daß die besagte Säure unter Freisetzung des Glycidylesters und Regenerierung des Harzes in der Form OH⁻ an den Ringen fixiert wird.

2. Herstellungsverfahren nach Anspruch 1, dadurch gekennzeichnet, daß die Reaktion in wässerigem Medium oder in einem den vorhandenen Verbindungen gegenüber inerten organischen Medium durchgeführt wird.

3. Herstellungsverfahren nach Anspruch 1, dadurch gekennzeichnet, daß die Reaktion in einem Überschuß von Carbonsäure durchgeführt wird.

4. Verfahren nach einem der Ansprüche 1, 2 oder 3, bei dem das Glycidol und eine Fettsäure zur Erzeugung eines Glycerids benutzt werden.

5. Verfahren nach Anspruch 4 zur Herstellung eines Monoglycerids, dadurch gekennzeichnet, daß man das Glycidol bzw. Glycidolderivat und die Fettsäure in einem aus polyoxygeniertem Äther bestehenden Lösungsmittel mischt.

6. Verfahren nach Anspruch 4 zur Herstellung von Glycerinmonooleat aus Glycidol und Oleinsaure, dadurch gekennzeichnet, daß man diese Verbindungen in einem Lösungsmittel der folgenden Gruppe mischt: 1.4-Dioxan, Äthylacetat, Aceton, Diglyme oder Triglyme.

7. Verfahren nach Anspruch 5 zur Herstellung eines Glycerinmonostearats aus Glycidol und Stearinsäure, dadurch gekennzeichnet, daß man diese Verbindungen in einem aus 1.4-Dioxan oder Diglyme oder Triglyme bestehenden Lösungsmittel mischt.

8. Verfahren nach Anspruch 5 zur Herstellung eines Glycerinmonoundecylenoats aus Glycidol und Undecylensäure, dadurch gekennzeichnet, daß man diese Verbindungen in einem aus 1.4-Dioxan oder Diglyme oder Triglyme bestehenden Lösungsmittel mischt.

9. Verfahren nach Anspruch 4 zur Herstellung eines Diglycerids oder Triglycerids, dadurch gekennzeichnet, daß man das Glycidol bzw. Glycidolderivat mit der Fettsäure in einem Kohlenwasserstofflösungsmittel mischt.

10. Verfahren nach Anspruch 9 zur Herstellung von Glycerindioleat und -trioleat aus Glycidol und Oleinsäure, dadurch gekennzeichnet, daß man diese Verbindungen in einem aus Hexan, Toluol oder Benzol bestehenden Lösungsmittel mischt.

11. Verfahren nach Anspruch 9 zur Herstellung von Glycerindistearat und -tristearat aus Glycidol und Stearinsäure, dadurch gekennzeichnet, daß man diese Verbindungen in einem aus Hexan, Toluol oder Benzol bestehenden Lösungsmittel mischt.

12. Verfahren nach einem der Ansprüche 1, 2 oder 3, dadurch gekennzeichnet, daß man zur Herstellung eines Glycerinfuranacrylats von Glycidol und einer Acrylfuransäure Gebrauch macht.

13. Verfahren nach Anspruch 1, 2 oder 3, bei dem man zur Herstellung eines verätherten Glycidylesters von einem Glycidoläther und einer Fettsäure Gebrauch macht.

14. Verfahren nach Anspruch 2, dadurch gekennzeichnet, daß man je Mol Glycidol bzw. Glycidolderivat im wesentlichen ein Mol Carbonsäure hinzufügt.

15. Verfahren nach einem der Ansprüche 2 oder 14, dadurch gekennzeichnet, daß man das Glycidol bzw. Glycidolderivat mit der Carbonsäure in wässerigem Medium oder inertem organischem Medium mischt, so daß die Konzentration jeder dieser Verbindungen zwischen 0,1 und 1 Mol Verbindung je Liter Medium beträgt.

16. Verfahren nach Anspruch 3, dadurch gekennzeichnet, daß man das Glycidol bzw. Glycidolderivat mit der Carbonsäure so mischt, daß die Konzentration des Glycidols bzw. Glycidolderivats zwischen 0,2 und 0,7 Mol Verbindung je Liter Medium beträgt.

17. Verfahren nach einem der Ansprüche 1 bis 16, dadurch gekennzeichnet, daß man das anionische Harz so vorsieht, daß die Anzahl aktiver Stellen OH⁻ ausgedrückt in Milliäquivalenten OH je Millimol Glycidol bzw. Glycidolderivat zwischen O,2 und 1 beträgt.

18. Verfahren nach Anspruch 17, dadurch gekennzeichnet, daß von einem anionischen Harz Gebrauch gemacht wird, dessen Kapazität zwischen 0,8 und 3 Milliäquivalenten OH je Gramm beträgt.

19. Verfahren nach Anspruch 17, dadurch gekennzeichnet, daß man von einem anionischen Harz der folgenden Gruppe Gebrauch macht: "Lewatit MP 500"-Harz der Firma "Bayer", "Amberlyst A 26" -Harz der Firma "Rohm and Haas".

20. Verfahren nach einem der Ansprüche 1 bis 19, durchgeführt bei einer Temperatur zwischen 20° C und 70° C.

21. Verfahren nach einem der Ansprüche 1 bis 20, das diskontinuierlich in einem Reaktionsgefäß durchgeführt wird, wobei man das Harz und die Lösung in dem Reaktionsgefäß mischt und das Harz sowie den gewonnenen Ester durch Filtrieren abscheidet.

22. Verfahren nach einem der Ansprüche 1 bis 20, das kontinuierlich in einer Reaktionssäule durchgeführt wird, wobei man Perkolieren der Lösung durch eine in der Reaktionssäule angeordnete Harzschicht bewirkt.

23. Herstellungsverfahren nach einem der Ansprüche 21 oder 22, wobei das Glycidol bzw. Glycidolderivat in einer Vorstufe in situ innerhalb des Reaktionsgefäßes bzw. der Reaktionssäule hergestellt wird, indem man in wässerigem Medium Deshydrohalogenierung eines Monohalohydrins in Gegenwart des anionischen Harzes bewirkt.

24. Herstellungsverfahren nach Anspruch 23, bei dem man in der ersten Stufe α-Monochlorhydrin benutzt.
